# EUROPÄISCHE PATENTSCHRIFT

(11) **EP 0 431 452 B1**
(45) Veröffentlichungstag und Bekanntmachung des Hinweises auf die Patenterteilung: **05.10.1994**
(21) Anmeldenummer: 90122703.3
(22) Anmeldetag: 28.11.1990
(51) Int. Cl.: A61K 6/083, A61K 6/00

(54) **Verfahren zur optischen Unterscheidung eines Dentalwerkstoffes sowie Dentalwerkstoff dafür**
Method for the optical distinction of dental materials and material used in this method
Procédé et distinction optique d'un matériau dentaire et matériau utilisé dans ce procédé

(30) Priorität: 02.12.1989 DE 3939998
(43) Veröffentlichungstag der Anmeldung: 12.06.1991
(73) Patentinhaber: IVOCLAR AG, FL-9494 Schaan (LI)
(72) Erfinder: Rheinberger, Volker, Dr., FL-9490 Vaduz (LI); Salz, Ulrich, Dr., W-8995 Weissenberg (DE); Ott, Gilbert, FL-9485 Nendeln (LI)
(74) Vertreter: UEXKÜLL & STOLBERG

(56) Entgegenhaltungen:
- EP-A- 0 375 161
- CH-A- 472 884
- GB-A- 2 190 917
- US-A- 4 600 389

## Beschreibung

Die Erfindung betrifft ein Verfahren zur optischen Unterscheidung eines auf ein Substrat aufgebrachten Dentalwerkstoffes von natürlichen Zähnen, künstlichen Zähnen oder Teilen davon sowie einen Dentalwerkstoff zur Verwendung in diesem Verfahren.

Verfahren zur optischen Unterscheidung eines Dentalwerkstoffes von natürlichen Zähnen sind bekannt. So wird in der US-PS 4 600 389 ein Verfahren beschrieben, bei dem fluoreszierende, in Mikroperlen eingearbeitete Lanthanidenverbindungen Dentalmaterialien zugesetzt werden und diese Dentalmaterialien anschließend mit der UV-Strahlung einer Quecksilberdampflampe mit einer Wellenlänge von 366 nm zur Fluoreszenz angeregt werden. Das dabei auftretende Fluoreszenzlicht zeigt je nach Art der eingesetzten Lanthanidenverbindung eine rote bzw. grüne Färbung, wodurch eine hinreichende Unterscheidbarkeit gegenüber der gleichfalls auftretenden bläulich-weißen Fluoreszenz der natürlichen Zähne gegeben sein soll. Die Einarbeitung der Lanthanidenverbindungen in Mikroperlen ist erforderlich, um deren Verträglichkeit mit unterschiedlichen Dentalmaterialien zu gewährleisten und außerdem ein vorzeitiges Ausbleiben des Fluoreszenzeffektes zu verhindern, was ansonsten durch die chemische Zersetzung und/oder das Auswaschen der Lanthanidenverbindungen hervorgerufen würde.

In der GB-OS 2 190 917 werden stark gefärbte Schutzfilme für Zähne beschrieben, die zusätzlich zu herkömmlichen Farbstoffen zur Steigerung der Unterscheidbarkeit gegenüber der dentalen Umgebung fluoreszierende Pigmente enthalten können, die mit Licht einer Wellenlänge von 400 bis 600 nm zur Fluoreszenz angeregt werden. Die Schutzfilme sind ausschließlich zum kurzfristigen Verbleib auf dem Zahn vorgesehen, wo sie als kontrastreicher Hintergrund für eine nachfolgende Befüllung von Kavitäten mit zahnfarbenem Füllmaterial dienen.

Bei beiden vorstehend aufgeführten Verfahren bleiben jedoch die Störeinflüsse unberücksichtigt, die auf die starke Eigenfluoreszenz der natürlichen Zähne zurückzuführen sind. So reichen schon die geringen Anteile an nahem UV-Licht des Tageslichts aus, um den natürlichen Zahn zur Fluoreszenz anzuregen. Ein sicheres Auseinanderhalten der fluoreszierenden Dentalwerkstoffe von den natürlichen Zähnen ist gemäß der bekannten Verfahren damit erschwert. Zusätzlich zu der bläulich-weißen Fluoreszenz der natürlichen Zähne stellt das gemäß GB-OS 2 190 917 zur Fluoreszenzanregung verwendete Blaulicht einer Wellenlänge von 400 bis 600 nm eine Störquelle dar, die das einfache Auseinanderhalten des eingesetzten Dentalmaterials und der umgebenden natürlichen Zähne behindert.

Der Erfindung liegt demgemäß die Aufgabe zugrunde, ein Verfahren zur optischen Unterscheidung eines auf ein Substrat aufgebrachten Dentalwerkstoffes von natürlichen Zähnen, künstlichen Zähnen oder Teilen davon, bei dem der Dentalwerkstoff durch die Einarbeitung der Fluoreszenzstoffe im wesentlichen keine sichtbare Farbveränderung erfährt, die Fluoreszenzstoffe auch ohne Einarbeitung in Mikroperlen mit unterschiedlichen Dentalwerkstoffen verträglich und gegenüber chemischen und physikalischen Einflüssen stabil sind, und bei dem die Störeinflüsse durch die Eigenfluoreszenz des natürlichen Zahnes und durch das anregende Blaulicht im wesentlichen ausgeschaltet werden, sowie einen Dentalwerkstoff zur Verwendung in diesem Verfahren zur Verfügung zu stellen.

Diese Aufgabe wird durch das Verfahren gemäß Anspruch 1 sowie den Dentalwerkstoff gemäß der Ansprüche 2 bis 4 gelöst.

Das erfindungsgemäße Verfahren zur optischen Unterscheidung eines auf ein Substrat aufgebrachten Dentalwerkstoffes von natürlichen Zähnen, künstlichen Zähnen oder Teilen davon ist dadurch gekennzeichnet, daß man in den Dentalwerkstoff 0,00001 bis 1 Gewichtsprozent eines Fluoreszenzstoffes einarbeitet, der im Wellenlängenbereich von 360 bis 480 nm ein Absorptionsmaximum und im Wellenlängenbereich von 480 bis 600 nm ein Fluoreszenzmaximum aufweist, den auf das Substrat aufgebrachten Dentalwerkstoff mit einer Lichtquelle bestrahlt, die Licht mit einer Wellenlänge im Bereich von 360 bis 480 nm aussendet, und durch ein Lichtfilter betrachtet, das Licht mit einer Wellenlänge im Bereich von 360 bis 480 nm zumindest teilweise ausfiltert.

Die im erfindungsgemäßen Verfahren in einer Menge von 0,00001 bis 1 Gewichtsprozent, bevorzugt 0,001 bis 0,1 Gewichtsprozent, in den Dentalwerkstoff eingearbeiteten Fluoreszenzstoffe zeichnen sich dadurch aus, daß sie im Wellenlängenbereich von 360 bis 480 nm ein Absorptionsmaximum und im Wellenlängenbereich von 480 bis 600 nm ein Fluoreszenzmaximum aufweisen.

Bevorzugte Fluoreszenzstoffe sind Cumarin-Derivate, Phthalimid-Derivate, Fluoranthen-Derivate, Perylen-Derivate, Xanthen-Derivate, Thioxanthen-Derivate, Pyrano-benzopyrano-2,5-dion-Derivate, Pyrano-chinolin-2,5-Derivate, Pyrazolchinoxalin-Derivate, 2-Pyrano-isochinolin-3,6-dion-Derivate, Benzimidazo-benz-isochinolin-7-on-Derivate, Acridin-Derivate und Mischungen der zuvor genannten Derivate. Besonders bevorzugt sind als Cumarin-Derivate, z.B.
7-(Dimethylamino)-4-(trifluormethyl)cumarin,
7-Amino-4-(trifluormethyl)cumarin,
7-(Dimethylamino)-4-(trifluormethyl)cumarin,
7-(Ethylamino)-6-methyl-4-(trifluormethyl)cumarin,
2,3,6,7-Tetrahydro-9-(trifluormethyl)-1H,5H,11H-[1]benzopyrano [6,7,8-ij]-chinolizin-11-on,
6,7,8,9-Tetrahydro-4-(trifluormethyl)-2H-pyrano [3,2,-c]-chinolin-2-on,
7-(Diethylamino)-3-(1-methyl-1H-benzimidazol-2-yl)-2H-1-benzopyran-2-on,
3-(2-Benzimidazolyl)-7-(diethylamino)-cumarin,
10-Acetyl-2,3,6,7-tetrahydro-1H,5H,11H-[1]benzopyrano [6,7,8-ij)chinolizin-11-on,
3-(2-Benzothiazolyl)-7-(diethylamino)cumarin,
2,3,6,7-Tetrahydro-11-oxo-1H,5H,11H-[1]benzopyrano [6,7,8-ij]chinolizin-10-carbonsäure und
2,3,6,7-Tetrahydro-11-oxo-1H,5H,11H-[1]benzopyrano [6,7,8-ij]chinolizin-10-carbonsäureethylester,
als Phthalimid-Derivate, z.B. 4-Amino-N-methyl-phthalimid,
4-(Dimethylamino)-N-methyl-phthalimid und
4-(2H-naphthol[1,2-d]triazol-2-yl)-N-methyl-phthalimid,
als Fluoranthen-Derivate, z.B. Fluoranthen-2,3-dicarbonsäureanhydrid und
1-Methyl-fluoranthen-2,3-dicarbonsäureanhydrid,
als Perylen-Derivate, z.B. Perylen und
3,9-Perylendicarbonsäurediisobutylester,
als Xanthen-Derivate, z.B. 2,8-Dimethylnaphtho[3,2,1-kl]xanthen,
als Thioxanthen-Derivate, z.B.

Benzothioxanthen-3,4-dicarbonsäure-N-stearylimid,
als Pyrano-benzopyrano-2,5-dion-Derivate,
z.B. 3-(Benzthiazol-2-yl)-2H,5H-pyrano[3,2,c][1]benzopyran-2,5-dion,
3-(Benzimidazol-2-yl)-2H,5H-pyrano[3,2,c][1]benzopyran-2,5-dion,
8-Hydroxy-3-(benzimidazol-2-yl)-2H,5H-pyrano[3,2,c][1]benzopyran-2,5-dion,
8-(Dimethylamino)-3-phenyl-2H,5H-pyrano[3,2,c][1]benzopyran-2,5-dion,
8-(Dimethylamino)-3-(benzothiazol-2-yl)-2H,5H-pyrano [3,2,c][1]benzopyran-2,5-dion und
8-(Dimethylamino)-2,5-dioxo-2H,5H-pyrano[3,2,c][1]benzopyran-3-carbonsäureethylester,
als Pyrano-chinolin-2,5-dion-Derivate,
z.B. 8-(Dimethylamino)-3-phenyl-2H-pyrano[3,2-c] chinolin-2,5(6H)-dion,
6-Methyl-3-(benzthiazol-2-yl)-2H-pyrano[3,2-c]chinolin-2,5 (6H)-dion und
8-(Dimethylamino)-2H-pyrano[3,2-c]chinolin-2,5(6H)-dion-3-carbonsäureethylester,
als Pyrazolchinoxalin-Derivate,
z.B. 7-(Dimethylamino)-3-methyl-1-phenyl-1H-pyrazolo[3,4-b] chinoxalin und
7-(Dimethylamino)-3-ethyl-1-phenyl-1H-pyrazolo[3,4-b] chinoxalin,
als 2-Pyrano-isochinolin-3,6-dion-Derivate,
z.B.2-(2'-Benzthiazolyl)-5-methyl-pyrano[2,3-c] isochinolin-3,6-dion,
2-(2'-Benzthiazolyl)-5-methyl-pyrano[2,3-c]isochinolin-3,6-dion und
2-(2'-Benzthiazolyl)-pyrano[2,3-c]isochinolin-3,6-dion,
als Benzimidazo-benz-isochinolin-7-on-Derivate,
z.B. 7H-Benzimidazo[2,1-a]benz[de]-isochinolin-7-on, und
als Acridin-Derivate,
z.B. 3,6-Diamino-2,7-dimethylacridinhydrochlorid,
3,6-Diaminoacridinhydrochlorid und
6,9-Diamino-2-ethoxyacridinhydrochlorid.

Weitere besonders bevorzugte Fluoreszenzstoffe sind:
2,2'-Dihydroxy-1,1'-naphthalazin,
4-Dimethylaminochalcon,
3-Methoxybenzanthron,
das Natriumsalz der
6-Amino-2,3-dihydro-1,3-dioxo-2-p-tolyl-1H-benz[de]iso-chinolin-5-sulfonsäure,
Chlorotetracyclin und/oder
N-Salicyliden-4-dimethylaminoanilin.

Die erfindungsgemäß eingesetzten Fluoreszenzstoffe erweisen sich besonders bei der Einarbeitung in farblose oder zahnfarbene Dentalwerkstoffe als vorteilhaft, da sie zu im wesentlichen keinerlei sichtbarer Verfärbung bei diesen führen. Als Dentalwerkstoffe finden erfindungsgemäß insbesondere Spacer-Lacke, Ausblockmaterialien, Versiegelungslacke für Prothesen, Dentin-Schutzlacke, Fissuren-Versiegler, Komposite-Füllungsmaterialien, wie Molarenmaterial, Befestigungskunststoffe für Kronen und Inlays, und Zahnzemente Anwendung. Bei der Auswahl des jeweils einzusetzenden Dentalwerkstoffes ist jedoch zu berücksichtigen, daß weder der Dentalwerkstoff selbst noch eine seiner Komponenten zu einer Veränderung des Fluoreszenzstoffes führen, wodurch dieser möglicherweise seine Fähigkeit zur Fluoreszenz verlieren würde. Die verwendeten Fluoreszenzstoffe sind mit den unterschiedlichsten Dentalwerkstoffen verträglich und verlieren ihre Fähigkeit zur Fluoreszenz auch dann nicht, wenn der mit ihnen ausgestattete Dentalwerkstoff, wie zum Beispiel ein Füllungsmaterial, über einen längeren Zeitraum den chemischen und physikalischen Einflüssen ausgesetzt ist, die auf die natürlichen Zähne einwirken. Damit ist es auch nicht wie bei bekannten Verfahren erforderlich, die Fluoreszenzstoffe zur Erzielung einer annehmbaren Verträglichkeit mit den Dentalwerkstoffen und einer geeigneten chemischen und physikalischen Stabilität in Mikroperlen einzuarbeiten.

Als Substrate auf die der mit dem Fluoreszenzstoff ausgestattete Dentalwerkstoff aufgebracht wird, sind insbesondere natürliche Zähne, künstliche Zähne oder Teile von diesen vorgesehen.

Damit der fluoreszierende bevorzugt farblose oder zahnfarbene Dentalwerkstoff in einfacher Weise von natürlichen Zähnen, künstlichen Zähnen, deren Teilen oder anderen Dentalmaterialien unterschieden werden kann bzw. besser sichtbar ist, wird er nach Aufbringen auf das Substrat mit einer Lichtquelle bestrahlt, die Licht mit einer Wellenlänge im Bereich von 360 bis 480 nm aussendet, und durch ein Lichtfilter betrachtet, das Licht mit einer Wellenlänge im Bereich von 360 bis 480 nm zumindest teilweise bevorzugt im wesentlichen vollständig ausfiltert. Auf diese Weise werden die im erfindungsgemäßen Dentalwerkstoff vorhandenen Fluoreszenzstoffe zur Emission von Fluoreszenzlicht angeregt, wodurch die Dentalwerkstoffe leicht von den sie umgebenden natürlichen Zähnen oder künstlichen Zähnen unterschieden werden können. Die bei bekannten Verfahren in Kauf genommenen Störeinflüsse durch Reflexionen des anregenden Lichtes und durch das im Wellenlängenbereich von 350 bis 480 nm liegende Fluoreszenzlicht der natürlichen Zähne werden erfindungsgemäß in einfacher Weise dadurch beseitigt, daß die störenden Lichtsorten mit einem Lichtfilter für Licht einer Wellenlänge von 360 bis 480 nm ausgefiltert werden. Besonders vorteilhaft ist, daß die erforderliche Lichtquelle und das erforderliche Lichtfilter den üblicherweise mit Dentalwerkstoffen befaßten Personen, wie z.B. Zahntechnikern oder Zahnärzten, bereits in Form von herkömmlichen Blaulichtlampen mit deren Hilfe zum Beispiel Kunststofffüllungen gehärtet werden und gängigen Orange-Filtern zum Schutz gegen dieses Blaulicht zur Verfügung stehen. Besonders bevorzugte Lichtquellen sind handelsübliche Blaulicht-Polymerisationslampen und besonders bevorzugte Lichtfilter sind zum Beispiel die von der Firma Röhm, Deutschland, vertriebenen Lichtfilter Plexiglas Orange 478 und Plexiglas Gelb 303. Die Anschaffung von besonderen UV-Lichtquellen, wie sie zur Durchführung von bekannten Verfahren erforderlich ist, entfällt damit.

Obwohl die erfindungsgemäßen Dentalwerkstoffe keine wesentliche Einfärbung durch die in ihnen eingearbeiteten Fluoreszenzstoffe erfahren, sind sie aufgrund des bei Anregung emittierten Fluoreszenzlichtes im Wellenlängenbereich von 480 bis 600 nm leicht von den sie umgebenden natürlichen Zähnen oder künstlichen Zähnen unterscheidbar und können damit sehr genau aufgetragen werden, wobei Überschüsse deutlich erkennbar sind. Bevorzugt finden das erfindungsgemäße Verfahren und die erfindungsgemäßen Dentalwerkstoffe immer dann Anwendung, wenn aus ästhetischen Gesichtspunkten bei Tageslicht farblose oder zahnfarbene Dentalwerkstoffe eingesetzt werden sollen, die aber dennoch von natürlichen Zähnen, künstlichen Zähnen, Teilen dieser und anderen Dentalmaterialien, auch über einen längeren Zeitraum hinweg, unterschieden werden können.

Die Erfindung wird in den folgenden Beispielen näher erläutert.

### Beispiel 1

### Spacer-Lack für Stümpfe

Herkömmliche Spacer-Lacke sind stark eingefärbt, damit sie genau auf einen Modellstumpf aufgetragen werden können. Diese Eigenfärbung des Lacks stört jedoch nach dem Aufbringen von Jacket-Kronen auf den Stumpf, da der Lack durch die Krone hindurch scheint und damit die Farbe der Krone verändert.

Es wurde ein Spacer-Lack durch Lösen von 50 g Elastosil E41 der Firma Wacker, Deutschland, und 50 mg des Fluoreszenzstoffes 7-Dimethylamino-4-trifluormethylcumarin in 60 g Toluol hergestellt. Der Spacer-Lack erwies sich selbst in dicken Schichten bei Tageslicht als praktisch farblos. Bei Bestrahlung mit dem Baulicht einer handelsüblichen Polymerisationslampe für lichthärtende Komposite und Betrachtung durch die von der Firma Röhm, Deutschland, vertriebenen Lichtfilter Plexiglas Orange 478 oder Plexiglas Gelb 303 oder auch durch eine handelsübliche Polymerisationslichtschutzbrille, wie sie häufig von Zahntechnikern oder Zahnärzten verwendet wird, war der Lack selbst in dünnen Schichten durch eine leuchtend gelb-grüne Farbe erkennbar.

### Beispiel 2

### Ausblockmaterial zum Ausblocken von Unterschnitten bei Modellen von Zahnpräparationen wie Kronenstümpfen oder Inlay-Kavitäten

Handelsübliche Ausblockmaterialien sind meistens intensiv eingefärbt, um eine gezielte Applikation zu ermöglichen. Diese intensive Eigenfarbe stört allerdings besonders bei Kunststoff- oder Keramik-Inlays sowie bei Jacket-Kronen, da sie bei der fertigen Arbeit durch das Inlay bzw. die Krone hindurchscheint und damit zu einer Farbveränderung führt.

Es wurde ein Ausblockmaterial durch Mischen von gleichen Teilen Basis- und Katalysator-Paste hergestellt.

Die Katalysator-Paste wurde hergestellt, indem 1,0 g Dibenzoylperoxid und 0,02 g Hydrochinon in 40 g Triethylenglykoldimethacrylat und 60 g bis-Phenol A-diglycidylmethacrylat gelöst wurden und diese Lösung in einem Mörser in 75 g silanisiertes Aerosil OX50 der Firma Degussa eingeknetet wurde.

Die dazugehörige Basis-Paste wurde analog hergestellt, wobei aber anstelle des Dibenzoylperoxids und des Hydrochinons 1,0 g N,N-Diethanol-p-toluidin und 0,1 g des Fluoreszenzstoffes 7-Dimethylamino-4-trifluormethylcumarin eingesetzt wurden. Die Topfzeit des Ausblockmaterials konnte durch Zugabe von Dibenzoylperoxid oder Hydrochinon auf einen Wert von 3 bis 4 Minuten eingestellt werden.

Das auf diese Weise erhaltene Ausblockmaterial hatte keine intensive Eigenfärbung. Dennoch konnte es wie in Beispiel 1 beschrieben, mit Hilfe einer Polymersationslampe und eines Filters gut sichtbar gemacht werden, wodurch eine gezielte Applikation möglich war.

### Beispiel 3

### Versiegelungslack für Prothesen

In einen aus 50 g Methylmethacrylat, 50 g Dipentaeritrytolmonohydroxypentaacrylat und 1,5 g Irgacure 651 der Firma Ciba-Geigy bestehenden Versiegelungslack für Prothesen wurden 5 mg des Fluoreszenzstoffes 3,9-Perylencarbonsäurediisobutylester gelöst.

Der auf diese Weise hergestellte Versiegelungslack erwies sich als praktisch farblos. Nachdem der Lack auf die Prothese aufgetragen worden war, konnte er jedoch, wie in Beispiel 1 beschrieben, mit Hilfe einer Polymerisationslampe und eines Filters sichtbar gemacht werden. Auf diese Weise konnte die aufgebrachte Lackschicht auf Fehlstellen und dünne bzw. ungleichmäßige Stellen überprüft werden, was besonders bei schwer zugänglichen Stellen wie den Interdentalbereichen oder den kritschen Bereichen beim Übergang vom Zahn zum Prothesenmaterial vorteilhaft war. Nach dieser Überprüfung wurde der Lack in einem Dental-Lichtpolymerisationsgerät gehärtet.

### Beispiel 4

### Dentin-Schutzlack

Es wurde ein fluoreszierender Dentin-Schutzlack durch Lösen von 15 mg Benzothioxanthen-3,4-dicarbonsäure-N-stearylimid und 30 g Vinapas C-305 der Firma Wacker in 200 g Diethylketon hergestellt.

Der Dentin-Schutzlack ließ sich, wie in Beispiel 1 beschrieben, mit Hilfe einer Polymerisationslampe und eines Filters sichtbar machen und leicht von natürlichen und künstlichen Zähnen unterscheiden, wodurch er gezielt appliziert und die vollständige Applikation überprüft werden konnte. Bei normalem Tageslicht hingegen war der Dentin-Schutzlack von natürlichen oder künstlichen Zähnen nicht unterscheidbar.

### Beispiel 5

### Fissuren-Versiegler

Bekannte Fissuren-Versiegler sind üblicherweise stark weiß pigmentiert, damit sei eindeutig vom natürlichen Zahn unterschieden werden können. Dies ist besonders bei der Applikation des Versieglers sowie für nachträgliche Kontrollen von Vorteil. Allerdings haben stark weiß pigmentierte Fissuren-Versiegler den Nachteil, daß sie sich farblich in ungewünschter Weise von den natürlichen Zähnen abheben und lichthärtende Produkte nur in dünnen Schichten polymerisiert werden können.

Es wurde ein fluoreszierender lichthärtender und zahnfarbener Fissuren-Versiegler hergestellt, indem in ein Monomer aus 0,003 g 3,9-Perylencarbonsäurediisobutylester, 0,3 g Campherchinon, 0,5 g N,N-3,5-Tetramethylanilin, 40 g Triethylenglykoldimethacrylat und 60 g bis-Phenol A-diglycidylether die für eine gewünschte Farbe erforderlichen Mengen an Titandioxid- und Eisenoxidpigmenten mit einem Dispergiergerät eingearbeitet wurden. Sämtliche Operationen wurden in einer Dunkelkammer durchgeführt.

Der auf diese Weise erhaltene zahnfarbene Fissuren-Versiegler konnte, wie in Beispiel 1 beschrieben, mit Hilfe einer üblichen Polymerisationslichtlampe und einem Lichtfilter aufgrund der Emission von gelb-grünem Fluoreszenzlicht deutlich von dem natürlichen Zahn unterschieden werden.

### Beispiel 6

### Komposite-Füllungsmaterial

Es wurde eine Monomerkomponente durch Lösen von 0,5 g N,N-3,5-Tetramethylanilin, 0,3 g Campherchinon und 0,1 g 7-Dimethylamino-4-trifluormethylcumarin in einem Gemisch aus 60 g bis-Phenol A-diclycidylether und 40 g Triethylenglykoldimethacrylat hergestellt. Gleichzeitig wurde ein Präpolymer hergestellt, indem 1 g Dibenzoylperoxid, 40 g silanisiertes Aerosil OX50, 30 g Dodecandioldimethacrylat und 30 g eines Trimethylhexamethylendiisocyanat-Dihydroxyethylmethacrylat-Adduktes gemischt wurden, die Mischung eine Stunde lang bei 120°C einer Hitzepolymerisation unterworfen und danach in einer Kugelmühle zu einer mittleren Korngröße von 30 µm gemahlen wurde. Zur Herstellung des Komposites wurden zuerst 21 g silanisiertes Aerosil OX50 der Firma Degussa und anschließend 50 g des Präpolymeren in 29 g der in einem Mörser vorgelegten Monomerkomponente eingearbeitet. Es wurde ein pastöses Komposite mit angenehmer, stopfbarer Konsistenz erhalten, wobei die gewünschte Farbe des Komposites durch Zugabe der erforderlichen Mengen an Farbpigmenten erzielt wurde. Außer der Herstellung des Präpolymeren wurden alle Arbeiten in einer Dunkelkammer durchgeführt.

Das auf diese Weise erhaltene zahnfarbene Komposite-Füllungsmaterial konnte im Gegensatz zu bekannten zahnfarbenen Komposite-Füllungsmaterialien, wie in Beispiel 1 beschrieben, zum natürlichen Zahn farblich konstrastierend dargestellt werden. Auf den Zahn überhängende Ränder waren auf diese Weise leicht erkennbar und damit entfernbar.

### Beispiel 7

### Befestigungskunststoff für Kronen und Inlays

Katalysator-Paste: Es wurde eine Monomerkomponente durch Lösen von 1,0 g Dibenzoylperoxid und 0,03 g Hydrochinon in einem Gemisch aus 30 g Triethylenglykoldimethacrylat und 70 g eines Trimethylhexamethylendiisocyanat-Dihydroxyethylmethacrylat-Addukts hergestellt. In 60 g der Monomerkomponente A wurden 40 g silanisiertes Aerosil OX50 eingerührt, wodurch eine Paste mit fließender Konsistenz erhalten wurde.

Basis-Paste: Durch Lösen von 1,0 g N,N-3,5-Tetramethylanilin, 0,6 g Campherchinon und 0,08 g 7-Dimethylamino-4-trifluormethylcumarin in einem Gemisch aus 30 g Triethylenglykoldimethacrylat und 70 g eines Trimethylhexamethylendiisocyanat-Dihydroxyethylmethacrylat-Adduktes hergestellt. In 60 g dieser Monomerkomponente wurden 40 g silanisiertes Aerosil OX50 und die zur Farbgebung erforderliche Pigmentmenge eingerührt. Alle Operationen zur Herstellung der Basis-Paste wurden in einer Dunkelkammer durchgeführt.

Durch Mischen von gleichen Teilen Basis- und Katalysator-Paste wurde die Selbsthärtung des Materials ausgelöst, wobei die Topfzeit 3 bis 4 Minuten betrug. Das Material konnte auch durch Bestrahlung mit Polymerisationslicht einer Wellenlänge von 400 bis 500 nm polymerisiert werden.

Der beschriebene Befestigungskunststoff erwies sich bei Tageslicht als zahnfarben. Zur Entfernung von Überschüssen nach der Zementierung von Kronen und Inlays konnte er jedoch, wie in Beispiel 1 beschrieben, von den natürlichen Zähnen bzw. den Kronen oder Inlays unterschieden werden.

## Patentansprüche

1. Verfahren zur optischen Unterscheidung eines auf ein Substrat aufgebrachten Dentalwerkstoffes von natürlichen Zähnen, künstlichen Zähnen oder Teilen davon, **dadurch gekennzeichnet**, daß man in den Dentalwerkstoff 0,00001 bis 1 Gew.% eines Fluoreszenzstoffes einarbeitet, der im Wellenlängenbereich von 360 bis 480 nm ein Absorptionsmaximum und im Wellenlängenbereich von 480 bis 600 nm ein Fluoreszenzmaximum aufweist, den auf das Substrat aufgebrachten Dentalwerkstoff mit einer Lichtquelle bestrahlt, die Licht mit einer Wellenlänge im Bereich von 360 bis 480 nm aussendet, und durch ein Lichtfilter betrachtet, das Licht mit einer Wellenlänge im Bereich von 360 bis 480 nm zumindest teilweise ausfiltert.

2. Dentalwerkstoff zur Verwendung in dem Verfahren nach Anspruch 1, **dadurch gekennzeichnet**, daß er Cumarin-Derivate, Phthalimid-Derivate, Fluoranthen-Derivate, Perylen-Derivate, Xanthen-Derivate, Thioxanthen-Derivate, Pyrano-benzopyrano-2,5-dion-Derivate, Pyrano-chinolin-2,5-Derivate, Pyrazolchinoxalin-Derivate, 2-Pyrano-isochinolin-3,6-dion-Derivate, Benzimidazo-benz-isochinolin-7-on-Derivate, Acridin-Derivate oder Mischungen der zuvor genannten Derivate als Fluoreszenzstoffe enthält.

3. Dentalwerkstoff nach Anspruch 2, **dadurch gekennzeichnet**, daß er als Cumarin-Derivate 7-(Dimethylamino)-4-(trifluormethyl)cumarin,
7-Amino-4-(trifluormethyl)cumarin,
7-(Dimethylamino)-4-(trifluormethyl)cumarin,
7-(Ethylamino)-6-methyl-4-(trifluormethyl)cumarin,
2,3,6,7-Tetrahydro-9-(trifluormethyl)-1H,5H,11H-[1]benzopyrano[6,7,8-ij]-chinolizin-11-on,
6,7,8,9-Tetrahydro-4-(trifluormethyl)-2H-pyrano [3,2,-c]-chinolin-2-on,
7-(Diethylamino)-3-(1-methyl-1H-benzimidazol-2-yl)-2H-1-benzopyran-2-on,
3(2-Benzimidazolyl)-7-(diethylamino)-cumarin,
10-Acetyl-2,3,6,7-tetrahydro-1H,5H,11H-[1]benzopyrano [6,7,8-ij)chinolizin-11-on,
3-(2-Benzothiazolyl)-7-(diethylamino)cumarin,
2,3,6,7-Tetrahydro-11-oxo-1H,5H,11H-[1]benzopyrano [6,7,8-ij]chinolizin-10-carbonsäure und/oder
2,3,6,7-Tetrahydro-11-oxo-1H,5H,11H-[1]benzopyrano [6,7,8-ij]chinolizin-10-carbonsäureethylester,
als Phthalimid-Derivate, 4-Amino-N-methyl-phthalimid,
4-(Dimethylamino)-N-methyl-phthalimid und/oder
4-(2H-naphthol[1,2-d]triazol-2-yl)-N-methyl-phthalimid,
als Fluoranthen-Derivate, Fluoranthen-2,3-dicarbon-säureanhydrid und/oder
1-Methyl-fluoranthen-2,3-dicarbonsäureanhydrid,
als Perylen-Derivate, Perylen und/oder
3,9-Perylendicarbonsäurediisobutylester,
als Xanthen-Derivate, 2,8-Dimethylnaphtho[3,2,1-kl]xanthen,
als Thioxanthen-Derivat,
Benzothioxanthen-3,4-dicarbonsäure-N-stearylimid,
als Pyrano-benzopyrano-2,5-dion-Derivate,
3-(Benzthiazol-2-yl)-2H,5H-pyrano[3,2,c][1]benzopyran-2,5-dion,
3-(Benzimidazol-2-yl)-2H,5H-pyrano[3,2,c][1]benzopyran-2,5-dion,
8-Hydroxy-3-(benzimidazol-2-yl)-2H,5H-pyrano[3,2,c][1]benzopyran-2,5-dion,
8-(Dimethylamino)-3-phenyl-2H,5H-pyrano[3,2,c][1]benzopyran-2,5-dion,
8-(Dimethylamino)-3-(benzothiazol-2-yl)-2H,5H-pyrano [3,2,c][1]benzopyran-2,5-dion und/oder
8-(Dimethylamino)-2,5-dioxo-2H,5H-pyrano[3,2,c][1]benzopyran-3-carbonsäureethylester,
als Pyrano-chinolin-2,5-dion-Derivate, 8-(Dimethylamino)-3-phenyl-2H-pyrano[3,2-c] chinolin-2,5(6H)-dion,
6-Methyl-3-(benzthiazol-2-yl)-2H-pyrano[3,2-c]chinolin-2,5 (6H)-dion und/oder
8-(Dimethylamino)-2H-pyrano[3,2-c]chinolin-2,5(6H)-dion-3-carbonsäureethylester,
als Pyrazolchinoxalin-Derivate,
7-(Dimethylamino)-3-methyl-1-phenyl-1H-pyrazolo[3,4-b] chinoxalin und/oder
7-(Dimethylamino)-3-ethyl-1-phenyl-1H-pyrazolo[3,4-b] chinoxalin,
als 2-Pyrano-isochinolin-3,6-dion-Derivate,
2-(2'-Benzthiazolyl)-5-methyl-pyrano[2,3-c] isochinolin-3,6-dion,
2-(2'-Benzthiazolyl)-5-methyl-pyrano[2,3-c]isochinolin-3,6-dion und/oder
2-(2'-Benzthiazolyl)-pyrano[2,3-c]isochinolin-3,6-dion,
als Benzimidazo-benz-isochinolin-7-on-Derivat,
7H-Benzimidazo[2,1-a]benz[de]-isochinolin-7-on, und
als Acridin-Derivate,
3,6-Diamino-2,7-dimethylacridinhydrochlorid, 3,6-Diaminoacridinhydrochlorid und/oder
6,9-Diamino-2-ethoxyacridinhydrochlorid enthält.

4. Dentalwerkstoff zur Verwendung in dem Verfahren nach Anspruch 1, **dadurch gekennzeichnet**, daß er 2,2'-Dihydroxy-1,1'-naphthalazin,
4-Dimethylaminochalcon,
3-Methoxybenzanthron,
das Natriumsalz der 6-Amino-2,3-dihydro-1,3-dioxo-2-p-tolyl-1H-benz[de]iso-chinolin-5-sulfonsäure,
Chlorotetracyclin und/oder
N-Salicyliden-4-dimethylaminoanilin als Fluoreszenzstoffe enthält.

## Claims

1. A method for the optical distinction of a dental material applied to a substrate from natural teeth, false teeth or parts thereof **characterized in that** 0.00001 to 1 wt.% of a fluorescent substance having an absorption maximum in the wavelength range from 360 to 480 nm and a fluorescence maximum in the wavelength range from 480 to 600 nm is incorporated in the dental material, the dental material applied to the substrate is irradiated with a light source emitting light of a wavelength in the range from 360 to 480 nm and then viewed through a light filter which filters out at least partially light having a wavelength in the range from 360 to 480 nm.

2. Dental material for use in the process according to claim 1 **characterized in that** it contains coumarin derivatives, phthalimide derivatives, fluoranthene derivatives, perylene derivatives, xanthene derivatives, thioxanthene derivatives, pyrano-benzopyrano-2,5-dione derivatives, pyrano-quinoline-2,5-derivatives, pyrazole quinoxaline derivatives, 2-pyrano-isoquinoline-3,6-dione derivatives, benzimidazo-benz-isoquinolin-7-one derivatives, acridine derivatives or mixtures of the aforementioned derivatives as fluorescent substances.

3. Dental material according to claim 2 **characterized in that** it contains as coumarin derivatives,
7-(dimethylamino)-4-(trifluoromethyl) coumarin,
7-amino-4-(trifluoromethyl) coumarin,
7-(dimethylamino)-4-(trifluoromethyl) coumarin,
7-(ethylamino)-6-methyl-4-(trifluoromethyl) coumarin,
2,3,6,7-tetrahydro-9-(trifluoromethyl)-1H,5H,11H-[1] benzopyrano [6,7,8-ij]-quinolizin-11-one,
6,7,8,9-tetrahydro-4-(trifluoromethyl)-2H-pyrano [3,2,-c]-quinolin-2-one,
7-(diethylamino)-3-(1-methyl-1H-benzimidazol-2-yl)-2H-1-benzopyran-2-one,
3-(2-benzimidazolyl)-7-(diethylamino) coumarin,
10-acetyl-2,3,6,7-tetrahydro-1H,5H,11H-[1] benzopyrano-[6,7,8-ij] quinolizin-11-one,
3-(2-benzothiazolyl)-7-(diethylamino) coumarin,
2,3,6,7-tetrahydro-11-oxo-1H,5H,11H-[1]benzopyrano[6,7,8-ij] quinolizine-10-carboxylic acid and/or
2,3,6,7-tetrahydro-11-oxo-1H,5H,11H-[1] benzopyrano-[6,7,8-ij] quinolizine-10-ethyl carboxylate,
as phthalimide derivatives,
4-amino-N-methyl phthalimide,
4-(dimethylamino)-N-methyl phthalimide and/or
4-(2H-naphthol[1,2-d]triazol-2-yl)-N-methyl phthalimide,
as fluoranthene derivatives,
fluoranthene-2,3-dicarboxylic acid anhydride and/or
1-methyl fluoranthene-2,3-dicarboxylic acid anhydride,
as perylene derivatives,
perylene and/or
3,9-perylene diisobutyl dicarboxylate,
as xanthene derivatives, 2,8-dimethylnaphtho[3,2,1-kl] xanthene,
as thioxanthene derivative, benzothioxanthene-3,4-dicarboxylic acid-N-stearylimide,
as pyrano-benzopyrano-2,5-dione derivatives,
3-(benzothiazol-2-yl)-2H,5H-pyrano[3,2,c][1]benzopyran-2,5-dione,
3-(benzimidazol-2-yl)-2H,5H-pyrano[3,2,c][1]benzopyran-2,5-dione,
8-hydroxy-3-(benzimidazol-2-yl)-2H,5H-pyrano-[3,2,c] [1]benzopyran-2,5-dione,
8-(dimethylamino)-3-phenyl-2H,5H-pyrano[3,2,c][1]benzopyran-2,5-dione,
8-(dimethylamino)-3-(benzothiazol-2-yl)-2H,5H-pyrano-[3,2,c][1]benzopyran-2,5-dione and/or
8-(dimethylamino)-2,5-dioxo-2H,5H-pyrano[3,2,c][1]benzopyran-3-ethyl carboxylate,
as pyranoquinoline-2,5-dione derivatives,
8-(dimethylamino)-3-phenyl-2H-pyrano[3,2,c]-quinoline-2,5(6H)-dione,
6-methyl-3-(benzothiazol-2-yl)-2H-pyrano[3,2-c] quinoline-2,5-(6H)-dione and/or
8-(dimethylamino)-2H-pyrano[3,2-c] quinoline-2,5-(6H)-dione-3-ethyl carboxylate,
as pyrazole quinoxaline derivatives,
7-(dimethylamino)-3-methyl-1-phenyl-1H-pyrazolo[3,4-b] quinoxaline and/or
7-(dimethylamino)-3-ethyl-1-phenyl-1H-pyrazolo[3,4-b] quinoxaline,
as 2-pyrano-isoquinoline-3,6-dione derivatives,
2-(2'-benzothiazolyl)-5-methyl-pyrano[2,3-c] isoquinoline-3,6-dione,
2-(2'-benzothiazolyl)-5-methyl-pyrano[2,3-c] isoquinoline-3,6-dione and/or
2-(2'-benzothiazolyl)-pyrano[2,3-c]isoquinoline-3,6-dione,
as benzimidazo-benz-isoquinolin-7-one derivative,
7H-benzimidazo[2,1-a]benz[de]-isoquinolin-7-one and
as acridine derivatives
3,6-diamino-2,7-dimethylacridine hydrochloride,
3,6-diaminoacridine hydrochloride and/or
6,9-diamino-2-ethoxyacridine hydrochloride.

4. Dental material for use in the process according to claim 1, **characterized in that** it contains as fluorescent substances,
2,2'-dihydroxy-1,1'-naphthalazine,
4-dimethyl-aminochalcone,
3-methoxybenzanthrone,
the sodium salt of 6-amino-2,3-dihydro-1,3-dioxo-2-p-tolyl-1H-benz[de]isoquinoline-5-sulphonic acid,
chlorotetracyclin and/or
N-salicylidene-4-dimethylaminoaniline.

## Revendications

1. Procédé de distinction optique d'un matériau dentaire, déposé sur un substrat, d'avec des dents naturelles, des dents artificielles ou des parties desdites dents, caractérisé en ce qu'on incorpore au matériau dentaire 0,00001 à 1 % en poids d'une substance fluorescente, qui présente un maximum d'absorption dans le domaine de longueurs d'onde 360-480 nm et un maximum de fluorescence dans le domaine de longueurs d'onde 480-600 nm, on irradie le matériau dentaire déposé sur le substrat avec une source de lumière qui émet de la lumière de longueur d'onde comprise entre 360 et 480 nm, et on l'observe à travers un filtre optique qui filtre au moins partiellement la lumière de longueur d'onde comprise entre 360 et 480 nm.

2. Matériau dentaire utilisable dans le procédé selon la revendication 1, caractérisé en ce qu'il contient, comme substances fluorescentes, des dérivés de coumarine, des dérivés de phtalimide, des dérivés de fluoranthène, des dérivés de pérylène, des dérivés de xanthène, des dérivés de thioxanthène, des dérivés de pyrano-benzopyrane-2,5-dione, des dérivés 2,5-pyranoquinoléiques, des dérivés de pyrazoquinoyaline, des dérivés de 2-pyrano-isoquinoléine-3,6-dione, des dérivés de benzimidazo-benzisoquinoléin-7-one, des dérivés d'acridine et des mélanges des dérivés précédemment mentionnés.

3. Matériau dentaire selon la revendication 2, caractérisé en ce qu'il contient
des dérivés de coumarine tels que:
7-(diméthylamino)-4-(trifluorométhyl)coumarine,
7-amino-4-(trifluorométhyl)coumarine,
7-(diéthylamino)-4-(trifluorométhyl)coumarine,
7-(éthylamino)-6-méthyl-4-(trifluorométhyl)coumarine,
2,3,6,7-tétrahydro-9-(trifluorométhyl)-1H,5H,11H-[1]benzopyrano[6,7,8-ij]quinolizin-11-one,
6,7,8,9-tétrahydro-4-(trifluorométhyl)-2H-pyrano[3,2-c]quinoléin-2-one,
7-(diéthylamino)-3-(1-méthyl-1H-benzimidazol-2-yl)-2H-1-benzopyran-2-one,
3-(benzimidazol-2-yl)-7-(diéthylamino)coumarine,
10-acétyl-2,3,6,7-tétrahydro-1H,5H,11H-[1]benzopyrano[6,7,8-ij]quinolizin-11-one,
3-(benzothiazol-2-yl)-7-(diéthylamino)coumarine,
acide 2,3,6,7-tétrahydro-11-oxo-1H,5H,11H-[1]benzopyrano[6,7,8-ij]quinolizin-10-carboxylique, et/ou
2,3,6,7-tétrahydro-11-oxo-1H,5H,11H-[1]benzopyrano[6,7,8-ij]quinolizin-10-carboxylate d'éthyle,
des dérivés de phtalimide tels que:
4-amino-N-méthylphtalimide,
4-(diméthylamino)-N-méthylphtalimide, et/ou
4-(2H-naphto[1,2-d]triazol-2-yl)-N-méthylphtalimide,
des dérivés de fluoranthène tels que:
anhydride d'acide fluoranthène-2,3-dicarboxylique, et/ou anhydride d'acide 1-méthylfluoranthène-2,3-dicarboxylique,
des dérivés de pérylène tels que le pérylène et
le 3,9-pérylènedicarboxylate de diisobutyle,
des dérivés de xanthène tels que le 2,8-diméthylnaphto[3,2,1-kl]xanthène,
des dérivés de thioxanthène tels que le N-stéarylimide d'acide benzothioxanthène-3,4-dicarboxylique,
des dérivés de pyrano-benzopyrane-2,5-dione tels que:
3-(benzothizol-2-yl)-2H,5H-pyrano[3,2-c][1]-benzopyrane-2,5-dione,
3-(benzimidazol-2-yl)-2H,5H-pyrano[3,2-c][1]-benzopyrane-2,5-dione,
8-hydroxy-3-(benzimidazol-2-yl)-2H,5H-pyrano[3,2-c][1]-benzopyrane-2,5-dione,
8-(diméthylamino)-3-phényl-2H,5H-pyrano[3,2-c][1]-benzopyrane-2,5-dione,
8-(diméthylamino)-3-(benzothiazol-2-yl)-2H,5H-pyrano[3,2-c][1]-benzopyrane-2,5-dione, et/ou
8-(diméthylamino)-2,5-dioxo-2H,5H-pyrano[3,2-c][1]-benzopyrane-3-carboxylate d'éthyle,
des dérivés de pyrano-quinoléine-2,5-dione tels que: 8-(diméthylamino)-3-phényl-2H-pyrano[3,2-c]quinoléine-2,5(6H)-dione,
6-Méthyl-3-(benzothiazol-2-yl)-2H-pyrano[3,2-c]quinoléine-2,5(6H)-dione, et/ou
8-(diméthylamino)-2H-pyrano[3,2-c]quinoléine-2,5(6H)-dione-3-carboxylate d'éthyle,
des dérivés de pyrazoquinoxaline tels que:
7-(diméthylamino)-3-méthyl-1-phényl-1H-pyrazolo[3,4-b]quinoxaline, et/ou
7-(diméthylamino)-3-éthyl-1-phényl-1H-pyrazolo[3,4-b]quinoxaline,
des dérivés de 2-pyrano-isoquinoléine-3,6-dione tels que:
2-(benzothiazol-2'-yl)-5-méthyl-pyrano[2,3-c]isoquinoléine-3,6-dione,
2-(benzothiazol-2'-yl)-5-éthyl-pyrano[2,3-c]isoquinoléine-3,6-dione, et/ou
2-(benzothiazol-2'-yl)-pyrano[2,3-c]isoquinoléine-3,6-dione,
un dérivé de benzimidazo-benzisoquinoléin-7-one tel que la 7H-benzimidazo[2,1-a]benz[de]isoquinoléin-7-one, et
des dérivés d'acridine tels que:
chlorhydrate de 3,6-diamino-2,7-diméthylacridine,
chlorhydrate de 3,6-diaminoacridine, et/ou
chlorhydrate de 6,9-diamino-2-éthoxyacridine.

4. Matériau dentaire utilisable dans le procédé selon la revendication 1, caractérisé en ce qu'il contient, comme substances fluorescentes, les substances suivantes:
2,2'-dihydroxy-1,1'-naphtalazine,
4-diméthylaminochalcone,
3-méthoxybenzanthrone,
6-amino-2,3-dihydro-1,3-dioxo-2-p-tolyl-1H-benz[de]
isoquinoléine-5-sulfonate de sodium,
chlorotétracycline, et/ou
N-salicylidène-4-diméthylaminoaniline.
